# EUROPEAN PATENT APPLICATION

(11) **EP 1 375 661 A1**
(43) Date of publication of application: **02.01.2004**
(21) Application number: 02077397.4
(22) Date of filing: 17.06.2002
(51) Int. Cl.: C12N 15/11, A61K 38/17, A61K 39/395, A61K 31/7088, A61P 35/00

(54) **The role of the N-myc - nm23H1/nm23H2 - cdc42 pathway in proliferation, differentiation and treatment of cancer**

(71) Applicant: ACADEMISCH ZIEKENHUIS BIJ DE UNIVERSITEIT VAN AMSTERDAM, 1105 AZ Amsterdam (NL)
(72) Inventor: Valentjn, Linda Johanna, 2151 TK Nieuw-Vennep (NL); Versteeg, Rogier, 3958 AA Amerongen (NL)
(74) Representative: Prins, Adrianus Willem

(57) **Abstract**

The invention relates to a method for the treatment of cancer by changing the intracellular activity of cdc42. The activity can be increased by treatment with small molecules, for instance treatment with non-hydrolyzable GTP-analogues of cdc-42. Also the activity can be increased by the inhibition of nm23-H1 and/or nm23-H2 which are factors which have an inhibitory effect on the expression and/or activity of cdc-42. It is also shown in the present invention that a decreasin the intracellular activity of cdc-42 has a beneficial effect.

Further embodiments of the invention are the compounds for use in such methods, pharmaceutical preparations comprising such compounds and use of such compounds for the preparation of a medicament for treatment of cancer.

## Description

The invention relates to the field of cancer, more specifically to chromosomal aberrations in tumor cells and the identification of new targets.

### Introduction

All cancer cells have chromosomal aberrations which cause these cells to behave as cancer cells thereby preventing differentiation. Although there is a large variety in clinically important carcinomas, it is assumed that the majority of these shares common features, which enables them to be recognized, but which also would contain targets for anti-cancer therapies. Neuroblastoma is a childhood tumor with a highly variable prognosis. Approximately 20% of neuroblastomas have N-myc amplification and these tumors follow a very aggressive course (Schwab et al. 1983, Seeger et al. 1985). Over-expression of transfected N-myc genes in neuroblastoma cell lines strongly increased proliferation rates (Bernards et al. 1986, Lutz et al. 1996). Transgenic mice over-expressing N-myc in neural crest-derived tissues showed a frequent development of neuroblastoma (Weis et al. 1997). Numerous comparable observations have implicated c-myc and L-myc in the pathogenesis of many other tumor types (Cole 1986, Marcu et al. 1992, Henrikson and Luscher 1996).

The myc-family members are transcription factors with a basic/helix-loop-helix/leucine zipper (bHLHzip) domain. Heterodimers of myc and MAX proteins bind to the E-box motive CACGTG and activate target gene transcription (Blackwood et al. 1992, Ma et al. 1993). The limited number of identified target genes thus far precluded the identification of myc downstream pathways. However, many experiments have suggested a role for myc genes in cell cycle control, metastasis, blocking of differentiation, apoptosis and proliferation rate (Henriksson and Luscher 1996, Schmidt 1999). The recently developed high throughput technologies - serial analysis of gene expression (SAGE) and micro-array analysis- led to the identification of many myc-regulated genes (Boon et al. 2001, Coller et al. 2000, Guo et al. 2000, Schuhmacher et al. 2001). We have used SAGE to identify all genes regulated by N-myc in neuroblastoma. Genes up-regulated by N-myc are involved in proliferation, transcription and translation (Boon et al 2001). N-myc down-regulated genes encode cytoskeletal proteins, extracellular matrix protein and their regulators (Valentijn et al. unpublished). With SAGE three genes were identified in the region on chromosomes 1p36 and 17q. Cdc42, located on chromosome 1p36 is down-regulated by N-myc (Valentijn unpublished) and nm23H1/NM23 and nm23-H2/NM23-H2 on chromosome 17q21 are up-regulated by N-myc (Godfried et al. 2002).

Rho GTPases modulate several cellular functions including cell-cell interactions, cell-matrix interactions, cell cycle progression and gene transcription (Hall 1998, Schmitz et al., 2000). The most studied family members are RhoA, Rac1 and Cdc42. The Rho-GTPases are regulated by GTPase-activating proteins (GAPs) and guanine nucleotide exchange factors (GEFs). A GAP inactivates through hydrolysis of the bound GTP to GDP and a GEF catalyzes the release of GDP and capture of a GTP. Functional studies of the Rho-GTPases have mainly been performed with the constitutively active and dominant negative mutants. These experiments implied a role in ras-dependent cell transformation. The expression of the active Cdc42-G12V mutant in Rati fibroblasts leads to anchorage-independent cell growth and proliferation in nude mice (Qiu et al. 1997). Activated Cdc42 also induced invasion of T-lymphoma cells into a fibroblast monolayer (Stam et al. 1998). Although the use of artificially activated mutants showed the involvement of Cdc42 in cell transformation, no mutations have been identified in tumors. The expression of Cdc42 protein is elevated in breast cancer and colon tumors (Fritz 1999).

Rho GTPases also have apparent roles in the actin cytoskeleton dynamics (Ridley 2001). A variety of receptors and down-stream pathways are involved in cytoskeletal rearrangments. Cdc42 induces filopodia formation in fibroblasts (Kozma et al, 1995). In line with the formation of filopodia, Cdc42 is also involved in neurite outgrowth. In murine N1E-115 cells grown on a laminin matrix, neuronal outgrowth was induced by serum-starvation or active Cdc42 (Sarner et al. 2000). The differentiation was not observed when cells were grown on fibronectin or poly-lysine, which indicates that the Cdc42-induced neurite outgrowth dependent of extracellular signalling and thus cell surface receptors..

The nm23 protein family consists of at least eight proteins encoding nucleoside diphosphate (NDP) kinases, the suppliers of nucleoside trophosphates (NTPs). Nm23-H1 was identified as suppressor of tumor metastasis in murine melanomas (Steeg et al. 1988). In human tumors among which breast cancer and melanoma, decreased expression was correlated with poor prognosis (Hennessy et al. 1991, Florenes et al. 1992). However, in neuroblastoma tumors elevated expression of nm23-H1 and H2 is associated with poor prognosis (Leone et al. 1993). The function of nm23 in tumor development is largly unknown. The identification of nm23 targets Rad and TIAM1 suggested a role as GAP (Zhu et al. 1999, Otsuki et al. 2001). Nm23-H1 negatively regulates TIAM1 and therefor inhibits Racland c-jun activation and thus might inhibit metastasis (Otsuki et al. 2001)

### Summary of the invention

We have now surprisingly found that cdc42 is a pivotal molecule in tumors and that up- or downregulation of this component can play a role in stopping cancerous outgrowth.
The invention therefore relates to a method for the treatment of cancer by changing the intracellular activity of cdc42. More specifically the invention relates to a method wherein the activity of cdc42 is increased. This can be achieved by administration of small molecules, said small molecules for instance being non-hydrolyzable GTP analogues of cdc-42.
Another embodiment of the invention is a method wherein the activity of cdc-42 is increased by inhibition of nm23-H1 and/or nm23-H2. This can be done by small molecules or by a protein which is able to bind to the nm23 protein. Such a protein can for example be an antibody or at least a binding part of an antibody.
Yet another embodiment of the invention is a method wherein the inhibition of nm23-H1 and/or nm23-H2 is established by inhibition of the expression of the genes coding for nm23-H1 and/or nm23-H2.
A following embodiment of the invention is a method wherein the activity of cdc-42 is decreased, which can be done by a small molecule or by inhibition of the expression of the gene coding for cdc-42.
According to the invention inhibition of expression of a gene can be accomplished by a double-stranded RNA through RNA interference or by an antisense nucleic acid. The length of such a dsRNA or antisense DNA is at least 12 nucleotides, more preferable 15 or more nucleotides, more preferably 15-25 nucleotides, most preferably 21 nucleotides.
The method according to the invention can be used for the treatment or prophylaxis of cancer especially for cancers selected from the group consisting essentially of neuroblastoma, colon carcinoma, gastric cancer, ovarian carcinoma, endometrial carcinoma, cervical carcinoma, sq. c. carcinoma in head and neck, non-SCLC, adenocarcinoma of the lung, osteosarcoma, retinoblastoma, childhood ALL, AML and non-Hodgkin lymphoma.

Also part of the invention is a compound useful for a method as described above. Thus, the invention comprises a compound for prophylactic and/or therapeutic treatment of an individual against cancer which is able to influence the intracellular activity of cdc-42.

One embodiment hereof is a compound which is able to increase the intracellular activity of cdc-42, whic preferably is a small molecule, such as a non-hydrolyzable GTP-analogue of cdc-42. Also part of the invention is a compound which is able to inhibit the activity of nm23-H1 and/or nm23-H2. Another embodiment of the invention is a compound which is able to decrease the intracellular activity of cdc-42. The compounds inhibiting the activity of the nm23 genes or cdc-42 can be a small molecule, a protein, more preferably an antibody or at least a binding part of an antibody. Another way of inhibiting the activity of nm23 genes or cdc-42 is use of a a nucleic acid, which can be a double-stranded RNA or an antisense nucleic acid. Such a nucleic acid comprises at least 12 nucleotides, more preferably 15 or more nucleotides, more preferably 15-25 nucleotides, most preferably 21 nucleotides. Accordingly, a compound according to the invention can be applied for the treatment of prophylaxis of cancer, wherein preferably the cancer is selected from the group consisting essentially of neuroblastoma, colon carcinoma, gastric cancer, ovarian carcinoma, endometrial carcinoma, cervical carcinoma, sq. c. carcinoma in head and neck, non-SCLC, adenocarcinoma of the lung, osteosarcoma, retinoblastoma, childhood ALL, AML and non-Hodgkin lymphoma.
Further part of the invention is a pharmaceutical preparation comprising a compound according to the invention and a pharmaceutically acceptable incipient.
Another part of the invention is the use of a compound according to the invention for the preparation of a medicament for the treatment of cancer.

### Legends of the figures

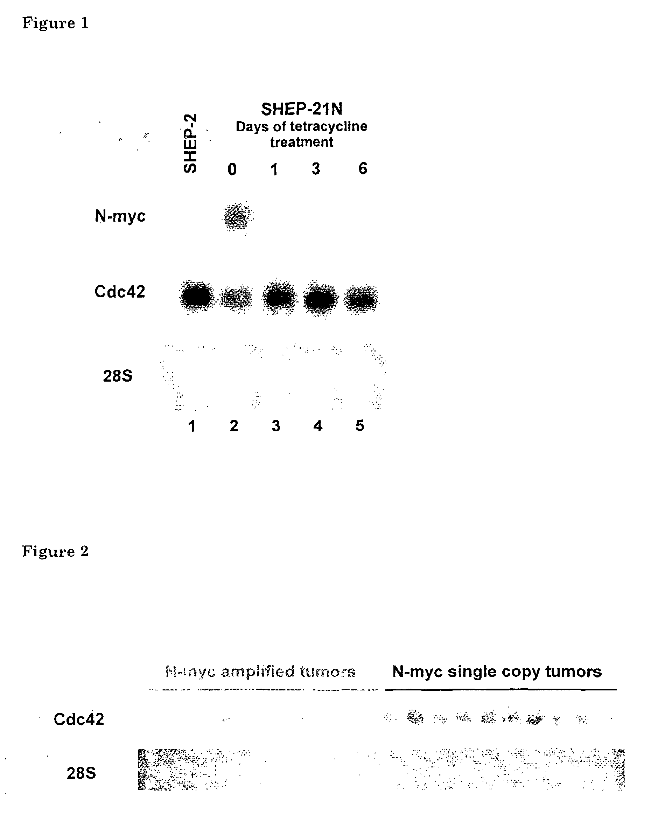

**Figure 1. Cdc42 is down-regulated by N-myc.** SHEP-21 N cells were grown in the absence (lane 2) or presence (lanes 3-5) of tetracycline. The mRNA expression of N-myc and Cdc42 was analyzed with Nothern blot analysis. Lane 1 contains total RNA of SHEP-2. The 28S ribosomal band is shown for loading control.
**Figure 2. Cdc42 expression in neuroblastoma tumors.** Northern blot analysis of Cdc42 expression in ten N-myc amplified and ten N-myc single copy tumors. The 28S ribosomal band is shown as loading control.

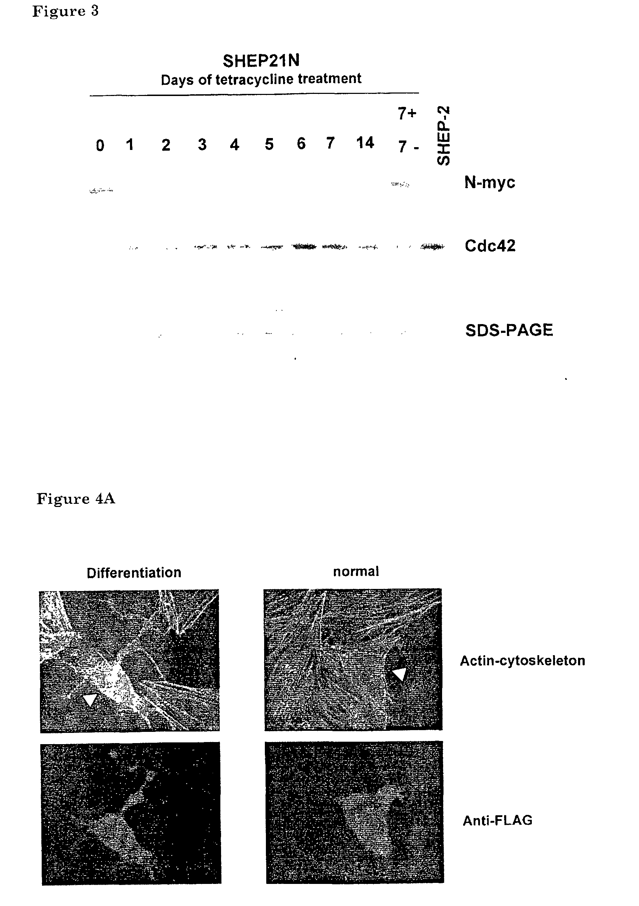

**Figure 3. Cdc42 protein levels are down-regulated by N-myc.** SHEP-21N cells were grown for several days (1-14) in the presence of tetracycline. After 7 days of treatment, SHEP-21N was grown 7 days without tetracycline (7+/7-). The last lane shows the SHEP-2 cell line. The expression of N-myc and Cdc42 protein was analyzed with Western blot analysis A part of the SDS-PAGE gel is shown as loading control.

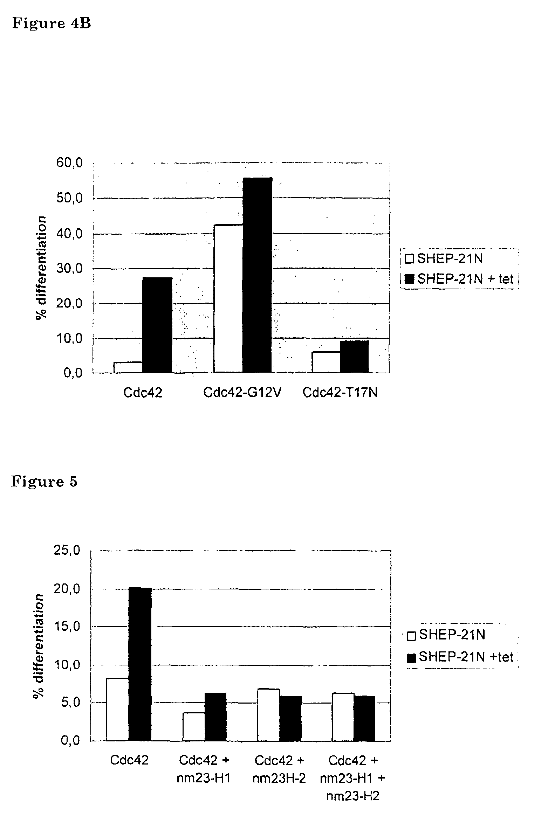

**Figure 4. Active Cdc42 induces neuronal differentiation in neuroblastoma cells.** SHEP-21N were grown without and with tetracycline (tet) and transfected with flag-tagged Cdc42, Cdc42-G12V or Cdc42-T17N. 40 hours after transfection cells were fixed and stained with an anti-FLAG antibody to identify transfected cells. More than 500 flag-positive cells were scored for neuronal differentiation. (A) Examples of differentiated and normal cells, The top panels show the staining of the actin cytoskeleton with Phalloidin-FITC. The arrowheads point to the Cdc42-flag expressing cells, as identified with the anti-FLAG antibody (lower panels). (B) Grafical representation of the percentage of differentiated cells after transfection with Cdc42flag, Cdc42-G12Vflag, or Cdc42-T17Nflag construct. The experiment was performed in culture medium with 1% FCS in the absence (white bars) or presence (black bars) of tetracycline.
**Figure 5. Neuronal differentiation by Cdc42 is inhibited by NM23-H1 and NM23-H2.** SHEP-21N were grown without (white bars) and with (black bars) tetracycline and transfected with flag-tagged Cdc42 and NM23-H1 and/or NM23-H2. 40 hours after transfection cells were fixed and stained with an antiflag antibody to identify transfected cells. Approximately 1000 flag-positive cells were scored for neuronal differentiation.

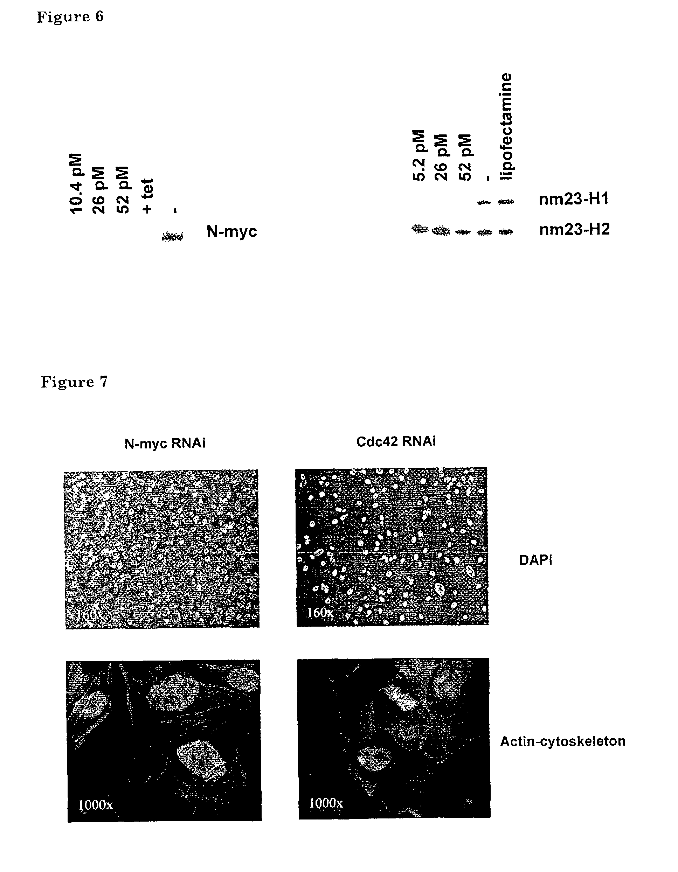

**Figure 6. Nmyc and nm23-H1 are down-regulated by RNA interference.** SHEP-21N cells were treated with 3 different concentrations of RNAi for N-myc, nm23-H1 or nm23-H2 as indicated above the lanes. Untreated (-) and only lipofectamine was used as negative controls. The SHEP-21N cells were treated with tetracycline (+ tet) as control for N-myc RNAi. 48 hours after transfection the efficiency of RNA interference was assay by Western blot using antibodies against N-myc, nm23-H1 and nm23H2 (indicated on the right).
**Figure 7. RNA interference of CDC42 inhibits cell division and actin remodeling.** An equal number of SHEP-21N cells were treated with RNAi for Nmyc (left) or Cdc42 (right). After 40 hours the cells were fixed, the cytoskeleton was stained with phallodin-TRITC (lower, red) and the nuclei were stained with DAPI (blue). Note the decrease in cell density after Cdc42 RNAi compared to N-myc RNAi (top panels).

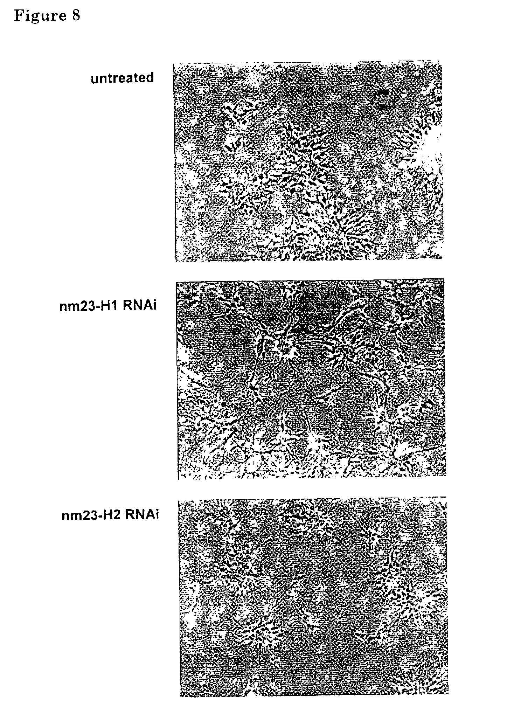

**Figure 8. RNA interference of nm23-H1 and nm23-H2 induces neurite outgrowth in S-JN-B1.** The S-JN-B1 cell line was treated with RNAi for nm23-H1 or nm23-H2. After 3 days the cells were photographed under phase-contrast microscopy.

### Detailed description of the invention

We started out with identification of downstream target genes of *N-myc.* For this we used an N-myc-transfected neuroblastoma cell line, generated by transfecting the SHEP cell-line, which has no N-myc amplification and expression, with a tetracyclin dependent N-myc expression vector (Lutz *et al.* 1996). Thus generated SHEP-21N cells have constitutive expression of N-myc which can be switched off by administration of tetracycline. From this cell-line and from a SHEP cell-line which was transfected with an empty vector (SHEP-2) SAGE libraries were constructed and sequenced. It appeared that one of the genes that was down-regulated as a result of N-myc expression was cdc-42.

In a following experiment it was established that cdc-42 and N-myc mRNA expression showed an inverse relation in ten tumors without and ten tumors with N-myc amplification.

Repression of cdc-42 by N-myc was further studied in the SHEP-21N cell line with ectopic N-myc expression. After switching off the N-myc expression with tetracycline, within 24 hours the cdc-42 expression increased, which proves that the expression of cdc-42 is, amongst others, regulated by N-myc.

The cdc-42 gene maps in the minimal region of chromosome band 1p35-36 that is consistently deleted in N-myc amplified neuroblastomas (Caron *et al.* 2001). Remarkably, deletion of one allele of this region (and cdc-42) is observed in about 90% of N-myc amplified neuroblastomas. The net result is that in these neuroblastomas cdc-42 expression is 50% reduced by deletion and the expression of the remaining allele is further down-regulated by N-myc.

Cdc-42 is a G-protein that is active when it is in the GTP-bound form and inactive in the GDP-bound state. The function of cdc-42 was studied in neuroblastomas. It appeared that after transfection of cells with a mutant cdc-42 which is permanently in the GTP-bound state, and thus active, the cells showed neuronal differentiation, which was not observed in untransfected cells. This was confirmed in a following experiment in which cells were transfected with a wild type cdc-42 or a dominant negative cdc-42 mutant that is permanently in the GDP-bound form. These cells, like untransfected cells, showed no differentiation. However, when both transfectants were treated with tetracycline, thereby switching off the inhibition by N-myc, the cells with the wild type cdc-42 started to differentiate, while the cells with the inactive form of cdc-42 were unable to induce differentiation. This proves that N-myc not only down-regulates the cdc42 mRNA expression level, but also can keep the cdc-42 in its inactive GDP-bound form.

Therefore, one embodiment of the invention is stimulating the activity of cdc-42 which would activate the cells to differentiate-into normal tissue cells. Such an activation can be achieved by administration of non-hydrolyzable GTP analogues or other activating small molecules. Small molecules in this context mean non-polymeric chemical compounds which have a specific pharmaceutical activity. Also envisaged is that systemic administration of small molecules which would be able to specifically increase the expression of cdc-42 or to specifically increase the amount of GTP-bound cdc-42, would be equally useful. It is now within the realm of a person skilled in the art to design a small molecule drug which would have the desired effect (Mauro M.J. *et al.,* J. Clin. Oncology 20(1): 325-334, 2002).

Cdc-42 is a G-protein and a member of the so-called Rho GTPases. These are regulated by GTPase-activating proteins (GAPs) and guanine nucleotide exchange factors (GEFs). Rho GTPases induce transcription regulation through intracellular signalling which activates transcription factors such as ELK, Max and ATF-2.The cycling between the inactive GDP-bound and active GTP-bound state of cdc-42 is regulated by GAPs and GEFs. The ability to induce neuronal differentiation as induced by the active form of cdc-42 can only be induced by the wild type cdc-42 if the inhibitory effect of N-myc is switched off. This indicates that N-myc expression prevents the transition of cdc-42 to its active form, probably by controlling a GAP or GEF of cdc-42. Previous experiments have shown that that nm23H and nm23H2 are upregulated by N-myc (Godfried et al., 2002). Furthermore, the nm23 genes map to a region on chromosome band 17q21 that is over-represented by a few copies in about 70% of neuroblastoma tumors (Caron et al., 1994; Bown et al. 1999). The net result is a strong over-expression of nm23 mRNAs in neuroblastoma (Godfried et al., 2002). Nm23 genes primarily function as dinucleotide kinases, but a possible role in GDP and GTP exchange has been postulated (Zhu et al., 1999; Otsuki et al., 2001), although never in connection with cdc-42.

We now show in this application that nm23 can control cdc-42 activity. This has been established in an experiment in which nm23H1 or nm23H2 are cotransfected to SHEP-21N cells without N-myc expression, which showed that this resulted in a reduction in differentiating cells. Further experiments pointed out that nm23H1 and nm23H2 functionally inactivate wild type cdc-42.

One embodiment of the invention now is to block the activity of nm23H1 and/or nm23H2 which will lead to an increase in active cdc-42 and thus to a decrease of cancerous cells because of neuronal differentiation. Blocking the activity of the nm23 activity can be effected in several ways.

A first possible way to block the effects of nm23 is by preventing its activity. This can be done by administering a compound which would bind to at least functional part of the nm23 protein. By a functional part of the nm23 protein is meant herein a part, which is, at least in part, involved in the GTPase activity or other functions of nm23. The binding component can be a small molecule which can be administered systemically. Design of such a drug can be achieved by a person skilled in the art (Mauro et al., 2002). In another embodiment the binding component is a protein, preferably an antibody, which can be administered systemically, e.g. by infusion into the bloodstream, or locally by injection. It is also envisaged that a cell could be transfected with a nucleotide sequence coding for such an antibody (or a functional part thereof) which upon expression would yield components which are able to bind to nm23.

The invention therefore provides a use according to the invention, wherein said compound is capable of specifically binding at least a functional part of a protein, which is involved in nm23 and therefore the cdc-42 activity. In one embodiment said compound comprises an antibody capable of specifically binding said at least functional part of a protein, or a functional part, derivative and/or analogue thereof. For instance, an antibody capable of specifically binding nm23 is suitable for a use of the invention.

A functional part of an antibody is defined as a part, which has the same kind of binding properties in kind, not necessarily in amount. By binding properties is meant the capability to specifically bind a target molecule. A functional derivative of an antibody is defined as an antibody, which has been altered such that the binding properties of said molecule are essentially the same in kind, not necessarily in amount. A derivative can be provided in many ways, for instance through conservative amino acid substitution.

A person skilled in the art is well able to generate analogous compounds of an antibody. This can for instance be done through screening of a phage display library. Such an analogue has essentially the same binding properties of said antibody in kind, not necessarily in amount.

Additionally, expression of a protein capable of influencing nm23 and therefore cdc-42 activity can be altered. For instance, expression of nm23 can be decreased. In that case, less protein will be present. If a protein capable of influencing the cdc-42 activity is less expressed, said cdc-42 activity, will as a result be less hampered. Expression of a protein can be altered with a protein capable of binding at least a functional part of a nucleic acid encoding said protein. Said binding to said functional part of a nucleic acid influences expression of said protein. Said binding, for instance, inhibits expression of said protein. The invention therefore provides a use according to the invention, wherein said compound comprises a protein capable of binding at least a functional part of a nucleic acid encoding a protein which is involved in nm23 and therefore CDC42 activity.

Expression of a protein can also be altered with a nucleic acid capable of binding at least a functional part of a nucleic acid encoding said protein. Said binding influences expression of said protein. The invention therefore provides a use according to the invention, wherein said compound comprises a nucleic acid capable of binding at least a functional part of a nucleic acid encoding a protein which is involved in nm23 and therefore cdc-42 activity. Preferably said compound comprises an antisense strand of at least a functional part of said nucleic acid, like for instance antisense nm23.

In terms of the invention, a functional part of a nucleic acid is defined as a part of said nucleic acid, at least 12 base pairs long, preferably at least 15-25 or more base pairs long, which is able to block the expression of a functional nm23 gene. Since the nucleotide sequence coding for nm23 is known (Dumas 1992) it is well within the power of a person skilled in the art to develop nucleic acids which would be useful in the present invention. In order to achieve specificity it is necessary to only block nm23-H1 and/or nm23-H2, which means that the nucleic acid used to block expression of these genes must be devised in such a way that other genes of the nm23 group should be able to still yield functional transcripts.

Inhibition of expression can also be obtained by a method of producing sequence-specific inhibition of gene expression by introducing a nucleotide construct capable of forming upon transcription one transcript or multiple transcripts, capable of folding into dsRNA. A process is provided for inhibition of expression of a homologous gene in a cell. The process comprises introduction of nucleotide constructs comprising nucleotide sequences homologous to nm23 sequences which may fold into partially or fully dsRNA molecules. When present in the target cells such nucleotide constructs inhibit expression of the nm23 gene(s) in a sequence-specific manner, in a process referred to as RNA silencing, RNA interference or RNAi (Ding S.W. 2000, Curr. Opin. Biotechnol. 11: 152-156). Inhibition of gene expression refers to the absence (or observable decrease) in the level of protein and/or mRNA product from the nm23 target gene(s). Specificity refers to the ability to inhibit the target gene without manifest effects on other (homologous) genes of the cell. It is emphasized that in the case of blocking expression of the nm23-H1 and/or nm23-H2 genes the dsRNA construct should be very specific, in order not to block other genes of the nm23 gene family.

Alternatively, the cell may be directly provided with a dsRNA molecule which shares a high homology with the nm23 gene. The RNA may comprise one or more strands of polymerised ribonucleotide. It may include modifications to either the phosphate-sugar backbone or the nucleoside. For example, the phosphodiester linkages of natural RNA may be modified to include at least one of a nitrogen or sulphur heteroatom. Modifications in RNA structure may be tailored to allow specific genetic inhibition while avoiding a general response which is generated in some cells by dsRNA. Likewise, bases may be modified to block the activity of adenosine deaminase. RNA may be produced enzymatically or by partial/total organic synthesis, any modified ribonucleotide can be introduced by *in vitro* enzymatic or organic synthesis.

The double-stranded structure may be formed by a single self-complementary RNA strand or by two complementary RNA strands. RNA duplex formation may be initiated either inside or outside the cell. The RNA may be introduced in an amount which allows delivery of at least one copy per cell. The RNA may be introduced directly into the cell (i.e. intracellularly) or introduced extracellularly into a cavity, interstitial space, into the circulation, or introduced orally.

A nucleotide construct which is able to form RNA containing a nucleotide sequence identical to a portion of the nm23 gene is preferred for inhibition. Also preferably the dsRNA will consist of 15-25 base pairs, more preferably 21 base pairs. The optimal sequences from the target RNA sequence to serve as target for the 21 nucleotide RNAi molecule can be determined with the primer3 program (Whitehead Institute for Biomedical Research, **http://www-genome.wi.mit.edu/genome.html**). A preferred target for the RNAi sequence is a stretch of 19 nucleotides preceding a double adenine, close to the translation start of the sequence coding for nm23.

Physical methods of introducing nucleic acids include injection of a solution containing the DNA or RNA, direct transfer of naked RNA or DNA or transfection by a virus carrying an expression construct. Other methods known in the art for nucleic acid transfer may be used, such as lipid-mediated carrier transport, chemical mediated transport, such as calcium phosphate, and the like.

The identification of the N-myc-Nm23-Cdc42 pathway in neuroblastoma implies that the pathway may be active in other tumors types as well. In general, pathways identified in specific tissues or tumor types often function in a broad range of normal and pathologic tissues. There are no indications that the pathway is restricted to neuroblastoma. In fact, a role for individual components of the pathway has been reported for many tumor types.

N-myc and c-myc are amplified and/or over expressed in a wide range of tumor types. N-myc and c-myc probably have identical functions. While c-myc and N-myc homozygous knockout mice are embryonic lethal, transgenic mice in which N-myc replaced c-myc showed a gross normal development, indicating that both proteins have largely overlapping functions (Malynn et al., 2000). We previously showed that all tested downstream target genes of N-myc are controlled by c-myc as well (Boon et al., 2001). Indeed Nm23H1 and Nm23H2 are up-regulated by N-myc as well as by c-myc (Godfried et al., 2002). Therefore, it is likely that many tumors with an activated myc oncogene do have an up-regulated expression of the Nm23-H1 and Nm23-H2 genes.

Nm23-H1 and/or Nm23-H2 indeed are found to be over expressed in many tumors. In some tumors, like breast cancer and melanoma, high expression is associated with a less metastatic phenotype and a better prognosis. Is these tumors, high Nm23 expression can be considered as anti-oncogenic and Nm23-H1 and Nm23-H2 clearly have another role than in neuroblastomas. However, in many other tumors, Nm23-H1 and Nm23-H2 expression is associated with aggressive tumor stages, a poor prognosis or with the transition from normal cells to tumor cells. This is reported for e.g. the following tumors: Neuroblastoma (Leone et al., 1993), Colon carcinoma (Berney et al., 2000), Ovarian carcinoma (Schneider et al., 2000), Retinoblastoma (Bardak et al., 2000), and Childhood ALL (Koomagi et al., 1998).

It is therefore likely that Nm23-H1 and Nm23-H2 perform a role in these tumors similar to their role in neuroblastoma. This is underscored by numerous reports for this group of tumors that imply activation of myc oncogenes, deletions of the 1p36 region including the Cdc42 locus and extra copies of the chromosome 17q region including the Nm23-H1 and Nm23-H2 genes. The therapeutic spectrum of an intervention in the Myc-Nm23-Cdc42 pathway is therefore likely to be wide and potentially includes a series of different tumor types.

As mentioned above, it is known that nm23 activity is upregulated in many tumors and is linked with aggressiveness of the carcinoma and a poor prognosis. This is reported for e.g. the tumors listed in table 1. Therefore, we submit that the present invention will be applicable over a wide range of both solid and non-solid tumors.

**Table 1.**

| ***List of tumors with high nm23 expression*** | |
|---|---|
| Tumor | Reference |
| | |
| Neuroblastoma | Leone et al., 1993 |
| Colon carcinoma | Berney et al., 2000 |
| Gastric cancer | Nesi et al. 2001 |
| Endometrial adenocarcinoma | Brustmann and Naude., 1999 |
| Ovarian carcinoma | Schneider et al., 2000 |
| Cervical carcinoma | Ravazoula et al., 2000 |
| Sq. c. carc. head and neck | Pavelic et al., 2000 |
| Non-SCLC | Volm et al., 1998 |
| Adenocarcinoma lung | Sato et al., 2000 |
| Osteosarcoma | Oda et al., 2000 |
| Retinoblastoma | Bardak et al., 2000 |
| Childhood ALL | Koomagi et al., 1998 |
| AML | Okabe-Kado et al., 1998 |
| non-Hodgkin lymphoma | Niitsu et al., 2001 |

Activation of Rho GTPases via overexpression or point mutation is thought to trigger cells to uncontrolled growth, inhibition of apoptosis and genetic instability (Bar-Sagi and Hall, 2000). Cdc-42 is located in the tumor suppression region on chromosome 1p36, which suggests that cdc-42 is a crucial gene in cancers and especially in neuroblastoma. As indicated above one of its main functions is in the regulation of the actin cytoskeleton dynamics. We now have shown that decreasing expression of cdc-42 has dramatic effects on cell growth. Treatment of cells expressing cdc-42 with a compound that inhibits functional expression of the gene (in this case RNAi-based inhibition) causes a decrease of cell growth which results in a 90% reduction of the number of cells as compared to untreated controls during a 48 hr test period. By staining it was revealed that in the absence of active cdc-42 cells are unable to rearrange the cytoskeleton after cell division, probably resulting in a cell cycle arrest.

Thus, in another embodiment of the invention cancerous outgrowth can be stopped by (further) decreasing the activity of cdc-42. Analogous to the inhibition of the nm23 activity, as described above, this can be achieved in various ways, basically by introduction of a component (e.g. a small molecule) which is able to bind cdc-42 or the nucleic acids which are necessary for the production of cdc-42. One of the preferred embodiments to achieve the inhibition of cdc-42 is to provide dsRNA which can bind to the endogenous mRNA which codes for cdc-42. This phenomenon is generally known as RNA interference or RNAi and is further explained above. Another preferred embodiment is the use of a small molecule for the inhibition of cdc-42 activity. Such a small molecule can be devised according to the method as described in Mauro et al., 2002).

Preferably inhibition of the activity of cdc-42 is a total inhibition, resulting in zero cdc-42 activity. It is however envisaged that an inhibition of more than 50%, more preferably 60%, more preferably 70%, more preferably 80%, more preferably 90% and most preferably more than 95% of the activity of cdc-42 as compared to the cdc-42 activity of wild type tumor cells, would be sufficient to stop growth of the cancer.

Both the increase of cdc-42 activity as well as the inhibition of cdc-42 activity can be used as an additional therapy in cancer patients. Thus, next to compounds having the above described effects on the activity of cdc-42 it would be possible to also apply more commonly known therapeutic anti-cancer treatments in addition to the methods of treatment and/or the pharmaceutical compounds according to the invention.

The following experiments are meant as an illustration to the invention and are not meant to limit the invention in any way.

### EXPERIMENTAL SECTION

### MATERIAL & METHODS

### Constructs

The primer sequences used for the construction of the plasmids are listed in table 2. The pCdc42flag plasmid was contructed by amplification of cDNA with primers Cdc42Efor and Cdc42Xrev. The amplified product was digested with EcoRI and XhoI and cloned into the *Eco*RI and *Xho*I sites of pCMV-Tag4 vector (Stratagene). The pCdc42G12Vflag and pCdc42T17Nflag constructs were produces by amplification of Cdc42 from vector pCdc42flag using primer T3 in combination with mutant primers CdcG12Vrev or CdcT17Nrev. The PCR products were digested with *Bam*HI and *Eco*RV and ligated in the *Bam*HI and *Eco*RV sites of pCdc42flag vector.

### Cell lines.

The SHEP-2 and SHEP-21N cell lines were cultured in RPMI with 10 % FCS, 2 mM glutamine, 50 U/ml penicillin, streptomycin. N-myc expression in SHEP-21N was repressed by adding 50 ng/ml tetracycline (Sigma). S-JN-B1 cell line was cultured in DMEM supplemented with 10 % FCS, 50 U/ml penicillin and streptomycin.

### Transient transfection and immunofluoresence microscopy.

For transient expression the cells were grown for 48 hours on glass slides in medium supplemented with 1 % FCS. 4 microgram plasmid DNA and 4 microgram DAC-30 (Eurogentec, Belgium) in 1 ml medium without FCS and antibiotics was incubated for 30 minutes at room temperature. In case of double transfections, 4 microgram of each plasmid was incubated with 8 microgram DAC-30. The DNA/DAC-30 mix was added 'drop-wise' to the cell culture. After 16 hours the medium was replaced by fresh medium with 1% FCS. 40 hours after transfection,the slides were fixed with 4% paraformaldehyde in phosphate-buffered saline (PBS) for 25 min. The cells were permeabilized with PBS/0.05% TritonX100. After washing 3 times with PBS/TX (PBS, 0.01% TritonX100) the slides were blocked for 30 min in Abdil (PBS, 0.01% TritonX100, 1% BSA) The Cdc42flag proteins were detected with mouse anti-flag M2 antibody (Stratagene) in 400 times diluted in Abdil for 30 min at room temperature. The slides were washed 4 times with PBS/TX. The secondary antibody, TRITC-conjugated anti-mouse (Sigma), 100 times diluted in Abdil was incubated for 30 min. After three times washing in PBS, the slides were rinsed with H₂O, dried and mounted in Vectashield (Vector laboratories) with DAPI.

### RNA interference

Cells were grown to 50 % confluency in a 6 cm plate. The sequences of the sense and antisense oligonucleotides (Isogen, the Netherlands) used for preparation of double stranded RNAi molecules are described in table 3. RNA interference was performed as described by Elbashir et al. (2001). The sequences of the single stranded RNA molecules for N-myc, Cdc42, nm23-H1 and nm23-H2 are listed in table 3. In short, 20 µM per single stranded RNA molecule in annealing buffer (0.1 M KAc, 30 mM HEPES/KOH pH7.4, 2 mM MgAc) was heated at 95 °C for 5 min, followed by a 1 hr incubation at 37 °C. The RNAi mixture was transfected serum free with 21 µl lipofectamine (Life Technonologies) as described by the manufacture. After 4 hours the medium was supplemented with 0.3 volume of culture medium with 30% FCS. The 20 µM RNAi in the annealing mixture results in a final concentration of 104 pM in the culture medium. The concentration in the annealing mixture was further reduced to 10, 5, 2 and 1 µM to assay the efficiency of RNA interference.

### Western blotting

Primary antibodies: anti-Nmyc (Pharmingen, 5000 times diluted), anti-nm23-H1 and anti-nm23-H2 (Kamiya biomedical company, MC-381 and MN-382). Westernblotting was performed according to standard procedures, secondary antibody anti-mouse linked to HRP (Amersham), detected with ECL KIT (Pharmacia)

### Northern Blot analysis

Total RNA (20 µg per lane) was separated on a 0.8% agarose gel in the presence of 6.7 % formaldehyde and blotted on Hybond N membranes (Amersham) in 10 x SSC. Hybridization was carried out overnight in 0.5 M NaHPO₄, pH 7.0, 7 % SDS, 1 mM EDTA at 65°C. Filters were washed in 40 mM NaHPO₄, 1% SDS at 65 °C. Probes were labeled by random priming of sequence-verified PCR products.

### EXAMPLE 1

### Cdc42 is down-regulated by N-myc

The N-myc oncogene is amplified in about 20% of neuroblastomas, which is associated with a poor clinical prognosis. To identify the downstream target genes of N-myc, we applied the SAGE technique to an N-myc-transfected neuroblastoma cell line. The SHEP cell line has no N-myc amplification and expression, nor c-myc expression. A tetracycline-dependent N-myc expression vector has been introduced into these cells, resulting in the SHEP-21N clone (Lutz et al. 1996). The SHEP-21N cells have constitutive exogenous N-myc expression that can be switched off by tetracycline. N-myc expression in the SHEP-21N cells was shown to increase the rate of cell division, shorten the G1 phase of the cell cycle and render the cells more susceptible to apoptotic triggers (Lutz et al. 1996; Fulda et al. 1999). SAGE libraries were constructed from SHEP-21N cells expressing N-myc and from SHEP-2 control cells. The SHEP-2 clone was transfected with the empty expression vector. We sequenced 44,301 SAGE tags from SHEP-2 and 34,426 SAGE tags from SHEP-21N. The transcripts corresponding to these tags were identified using the SAGEmap database (Lal et al., 1999) and our own tag assignment program (Caron et al., 2001). A series of genes down-regulated in the SHEP-21N cell line function in cytoskeleton regulation, cell-matrix interaction and extra-cellular matrix configuration. One of the genes identified as an N-myc downstream target is Cdc42 (Table 1).

The tag for Cdc42 has a frequency of 13 per 20,000 in SHEP2 and 3 per 20,000 in SHEP21N. Northern blot analysis confirmed the decreased expression of Cdc42 mRNA in SHEP-21N compared to SHEP-2 (figure 1, lane 2 versus 1, respectively). To test whether Cdc42 is indeed down-regulated by N-myc, we manipulated the N-myc expression in the SHEP-21N cell line. Tetracycline can switch off the ectopic N-myc expression in these cells. Within 24 hours after N-myc was switched off by tetracycline treatment, the Cdc42 expression increased (figure 1, compare lanes 2 and 3). The regulation of Cdc42 by N-myc was also investigated on Western blot. The Cdc42 protein level increased after N-myc was down-regulated (figure 3). After N-myc had been switched off during one week of tetracycline treatment, the N-myc gene was induced again, resulting in a decrease of Cdc42 (figure 3, time point 7+/7-). These data prove that the expression of Cdc42 mRNA is down regulated by N-myc, resulting in decreased Cdc42 protein levels.

Analysis of Cdc42 expression in other neuroblastoma SAGE libraries was in line with regulation of Cdc42 by N-myc. The expression of Cdc42 was also decreased in the libraries of N-myc amplified cell lines and tumors compared to the libraries of the N-myc single copy cases. The N-myc single copy cell line SK-N-Fi shows a frequency of 14.3 Cdc42 tags per 20,000 compared to 2.2 in the N-myc amplified IMR32 cell line (table 1). N-myc single copy tumors N165 and N52 have 5.8 and 3.1 tags, respectively, compared to 1.0 in the N-myc amplified N159 neuroblastoma tumor (table 1). The expression of Cdc42 mRNA was further analyzed by Northern blot analysis in a panel of neuroblastoma tumors. Cdc42 expression was reduced in the 10 tumors with N-myc amplification, compared to the N-myc single copy tumors (figure 2).

The Cdc42 gene maps in the minimal region of chromosome band 1p35-36 that is consistently deleted in N-myc amplified neuroblastomas (Caron et al 1995). Deletion of one allele of this region is observed in about 90% of N-myc amplified neuroblastomas. The net result is that Cdc42 expression in N-myc amplified neuroblastomas is 50% reduced by deletion of one allele and the expression of the remaining allele is further down-regulated by N-myc. We therefore analyzed whether Cdc42 can function as a tumor suppressor gene in neuroblastoma.

### EXAMPLE 2

### Cdc42 induces neuronal differentiation in SHEP-21N cells

Cdc42 is a G-protein that is active when in the GTP-bound state and inactive in the GDP-bound state. We investigated the role of Cdc42 in neuroblastoma by transient expression of the Cdc42 protein coupled to a C-terminal FLAG tag (Cdc42flag). We first transfected a mutant Cdc42 form that is permanently in the GTP-bound state (G12V mutant). The transiently transfected SHEP-21N cells were stained with an anti-flag antibody. The cells expressing the Cdc42-G12Vflag protein frequently showed neuronal differentiation (42%), as evident by long neurite extensions. This was not observed in untransfected cells (figure 4).

In addition, we transfected SHEP-21N with a wildtype Cdc42flag construct and with a FLAG construct of a dominant negative Cdc42 mutant (T17N) that is permanently in the GDP-form. Both the wild-type Cdc42 and the dominant negative. Cdc42-T17N did not show differentiation, suggesting that the wildtype Cdc42 protein is in SHEP-21N cells in the inactive GDP-bound state (figure 4). However, when N-myc was switched off with tetracycline, also wild type Cdc42flag-transfected cells displayed neuronal differentiation (28%), while the dominant negative Cdc42 construct remained inactive (figure 4b). This strongly indicated that N-myc not only down-regulates the Cdc42 mRNA expression level, but also can keep the Cdc42 protein in its inactive GDP-bound form.

### EXAMPLE 3

### nm23 regulates Cdc42

Cycling between the inactive GDP-bound and active GTP-bound state of Cdc42 is regulated by GAPs and GEFs. Neuronal differentiation as induced by Cdc42-G12V can only be induced by wild type Cdc42 when N-myc is switched off. This indicates the high N-myc expression prevents the function of Cdc42, probably by controlling a GAP or GEF of Cdc42. Previous experiments have shown that N-myc strongly up regulates the expression of the nm23H1 and nm23-H2 genes (Godfried et al., 2002). Furthermore, these genes map to a region on chromosome band 17q21 that is over-represented by a few copies in about 70% of neuroblastoma tumors (Caron et al. 1994; Bown et al. 1999) Together this results in a strong over-expression of nm23 mRNAs in neuroblastoma (Godfried et al., 2002). Nm23 genes primarily function as dinucleotide kinases, but a possible role in GDP and GTP exchange has been reported (Otsuki et al. 2001). However, nm23 was never described to function as a protein that can keep Cdc42 in the inactive GDP-bound state.

We performed the following experiments to analyze whether nm23 can control Cdc42 activity. Transient transfection of a wildtype Cdc42-flag construct into SHEP-21N cells resulted in 5% differentiation of the transfected cells (figure 5). When N-myc is switched of in these cells by tetracycline, the Cdc42flag-induced differentiation increased to 20% of the transfected cells. As N-myc down regulation causes down regulation of nm23-H1 and nm23-H2, we analyzed whether nm23-H1 or nm23-H2 can prevent the increase in differentiation by N-myc abrogation. We therefore transfected SHEP-21N cells with down-regulated N-myc expression with a flag-tagged Cdc42 wildtype construct and nm23-H1 and/or nm23-H2 expression vectors. The differentiation in either the nm23-H1 or nm23-H2 co-transfected cells was reduced to 5% (figure 5). The percentage of differentiated cells is not further reduced by transfection of both nm23-H1 and nm23-H2. Therefore, nm23-H1 and nm23-H2 have the same effect as N-myc expression in these cells: they prevent Cdc42-induced differentiation. As nm23-H1 and nm23-H2 are strongly up regulated by N-myc (Godfried et al., 2002), they probably mediate effect of N-myc.

Together, these data show that the Cdc42 function in neuroblastoma is blocked by a combination of N-myc over-expression and minor chromosomal aberrations. The nm23 genes on 17q21 are present in four to eight copies in most neuroblastomas. The mRNA expression of these copies is 6 to 10 times induced by N-myc, together leading to a very strong increase of nm23-H1 and nm23-H2 mRNA expression. The nm23-H1 and nm23-H2 proteins probably drive the Cdc42 proteins in an inactive GDP-bound state. Cdc42 is already hardly present in the cell, due to deletion of one allele and down-regulation of the mRNA expression of the remaining allele by N-myc. The differentiation-promoting function that we showed for Cdc42 is therefore completely blocked. This shows that Cdc42 probably functions as a neuroblastoma tumor suppressor gene.

### EXAMPLE 4

### RNA interference of nm23 gene expression

RNA interference is a powerful technique for mRNA specific and efficient reduction of intracellular proteins (Elbashir et al. 2001). A 21 nucleotide, double stranded RNA molecule directed ageinst the gene of interest is transfected in cells with almost 100% efficiency. The RNAi technique was assayed in the SHEP-21N cell line with RNAi molecules for N-myc, nm23-H1 and nm23-H2. Different concentrations were transfected and after 16 hours the protein levels were analyzed on Western blot. The N-myc RNA interference was compared with tetracycline treatment of SHEP-21N (figure 6). Transfection with 52 and 26 pM RNAi reduced N-myc to the same level of tetracycline treated cells. Further reduction to 10.4 pM N-myc RNAi still results in a reduction of N-myc protein, but slightly less effient.

RNA interference with RNAi molecules directed against nm23-H1 in SHEP-21N resulted in an efficient reduction of nm23-H1 protein. Also with a concentration of 5.2 pM, the nm23-H1 protein is still reduced to minimal levels (figure 6). Treatment of cells with nm23-H2 RNAi was less effient. A reduction was seen with 52 pM, but less efficient compared to nm23-H1.

In conclusion, using RNA interference we were able to switch of nm23-H1 and N-myc in SHEP-21N. The RNAi technique was also extended to other neuroblastoma cell lines, which resulted in similar results (data not shown).

### EXAMPLE 5

### Cdc42 RNA interference: Cdc42 is an essential gene

Neuroblastomas with N-myc amplification still have one Cdc42 allele. Mutation analysis in over 50 tumors and cell lines revealed no mutations in the Cdc42 coding region. The Cdc42 expression in N-myc amplified tumors suggests that reduced expression is a potential mechanism for tumorigenesis. We further decreased the expression of Cdc42 by RNA interference. Cdc42 specific RNAi was transfected in SHEP-21N cells. A strongly reduced expression of Cdc42 protein was confirmed with Western blot analysis (data not shown). Decrease of Cdc42 had dramatic effects on cell growth. Equal numbers of SHEP-21N cells were treated with Cdc42 iRNA or N-myc iRNA. After 40 hours, the number of Cdc42 iRNA-treated cells was 10-fold less than the N-myc iRNA-treated cells (figure 7). Staining of actin cytoskeleton revealed that in the absence of Cdc42, cells are incapable of re-arranging the cytoskeleton after cell division, which probably results in a cell cycle arrest. Thus Cdc42 is an essential gene for the growth and survival of neuroblastoma cells. Absence of Cdc42 causes cell cycle arrest. This explains why Cdc42 was not found to be mutated in neuroblastomas: a minimal amount of Cdc42 is required for cell division.

### EXAMPLE 6

### Inactivation of nm23H1 or nm23H2 induces differentiation of neuroblastoma cells.

The finding that nm23-H1 and nm23-H2 can prevent the Cdc42 induced differentiation in neuroblastoma cells predicts that down-regulation of these genes can induce differentiation of neuroblastoma cells. This was tested in the neuroblastoma cell line SJ-NB-1. This cell line has no N-myc amplification or expression, but extra copies of chromosome arm 17q are present in the cell and nm23-H1 and nm23-H2 are well expressed (Godfried et al. 2002). Cdc42 is also expressed in this cell line (data not shown). We inactivated nm23-H1 protein expression by treatment of the cells by RNA interference. Nm23-H1 specific RNAi was transfected in SJNB1, resulting in strong down regulation of nm23-H1 protein expression. Within 3 days, the cells showed an almost 100% differentiation, as evident by long neurite extensions (figure 8). This differentiation was followed by massive cell death. Also transfection with an RNAi specific for nm23-H2 induced general differentiation, but the neurite extensions of the cells were more limited (figure 8). This shows that under physiological concentrations of Cdc42, the down-regulation of nm23-H1 and nm23-H2 results in strong differentiation followed by massive cell death of neuroblastoma cells.

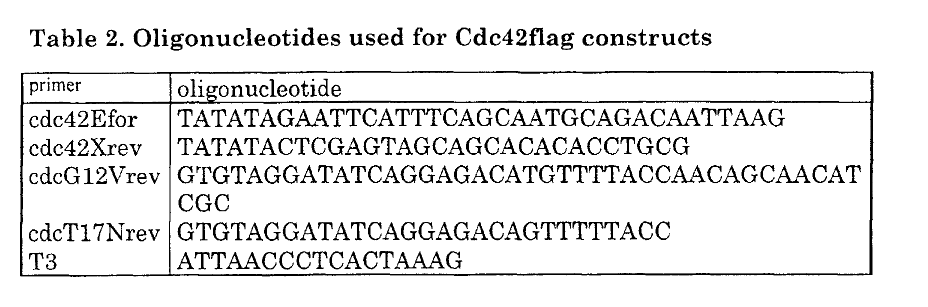

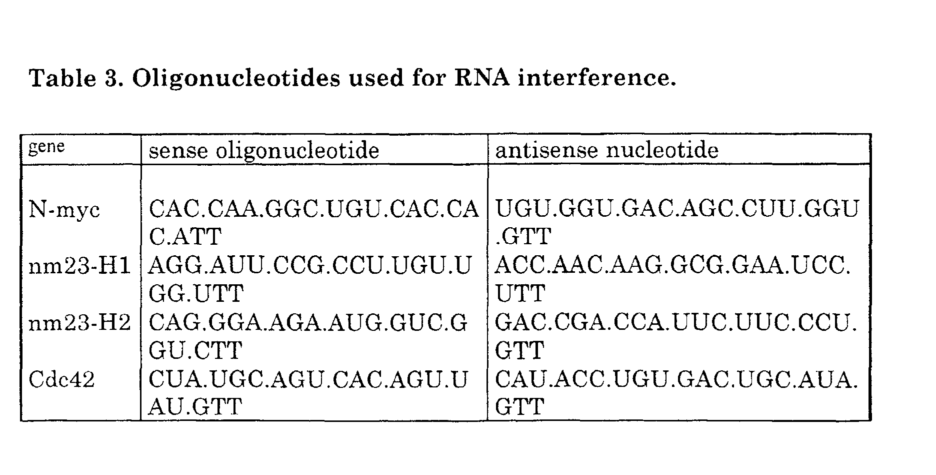

### References

Bar-Sagi D, Hall A (2000) Ras and Rho GTPases: a family reunion. Cell 103, 227-238.

Bardak Y, Cekic O, Ayhan A, Gunalp I, Bulay O (2000) Nucleoside diphosphate kinase (nm23 protein) expression in retinoblastoma. Ophthalmic Res 32, 73-78.

Bernards R, Dessain SK, Weinberg RA (1986). N-myc amplification causes down-modulation of MHC class I antigen expression in neuroblastoma. Cell 47, 667-674.

Berney CR, Fisher RJ, Yang J, Russell PJ, Crowe PJ (2000)Genomic alterations (LOH, MI) on chromosome 17q21-23 and prognosis of sporadic colorectal cancer. Int J Cancer 89, 1-7.

Blackwood EM, Kretzner L, Eisenman RN (1992). Myc and Max function as nucleoprotein complex. Curr. Opn. Genet. Dev. 2, 227-235.

Boon K, Caron HN, van Asperen R, Valentijn L, Hermus MC, van Sluis P, Roobeek I, Weis I, Voute PA, Schwab M, Versteeg R (2001) N-myc enhances the expression of a large set of genes functioning in ribosome biogenesis and protein synthesis. EMBO J 20, 1383-1393.

Bown N (2001) Neuroblastoma tumour genetics: clinical and biological aspects. J Clin Pathol 54, 897-910.

Bown N, Cotterill S, Lastowska M, O'Neill S, Pearson AD, Plantaz D, Meddeb M, Danglot G, Brinkschmidt C, Christiansen H, Laureys G, Speleman F (1999) Gain of chromosome arm 17q and adverse outcome in patients with neuroblastoma. N Engl J Med 340, 1954-1961.

Brustmann H, Naude S. (1999) Expression of nm23 in normal, hyperplastic and neoplastic endometrial tissues. Pathol Res Pract 195, 829-834

Caron H, van Sluis P, van Roy N, de Kraker J, Speleman F, Voute PA, Westerveld A, Slater R, Versteeg R (1994) Recurrent 1;17 translocations in human neuroblastoma reveal nonhomologous mitotic recombination during the S/G2 phase as a novel mechanism for loss of heterozygosity. Am J Hum Genet 55, 341-347.

Caron H, Peter M, van Sluis P, Speleman F, de Kraker J, Laureys G, Michon J, Brugieres L, Voute PA, Westerveld A (1995) Evidence for two tumour suppressor loci on chromosomal bands 1p35-36 involved in neuroblastoma: one probably imprinted, another associated with N-myc amplification. Hum Mol Genet 4, 535-539.

Caron H, van Schaik B, van der Mee M, Baas F, Riggins G, van Sluis P, Hermus MC, van Asperen R, Boon K, Voute PA, Heisterkamp S, van Kampen A, Versteeg R (2001) The human transcriptome map: clustering of highly expressed genes in chromosomal domains. Science 291, 1289-1292.

Cole MD (1986). The myc oncogene: its role in transformation and differentiation. Annu Rev Genet. 20, 361-84.

Coller HA, Grandori C, Tamayo P, Colbert T, Lander ES, Eisenman RN, Golub TR (2000) Expression analysis with oligonucleotide microarrays reveals that MYC regulates genes involved in growth, cell cycle, signaling, and adhesion. Proc Natl Acad Sci USA 97, 3260-3265.

Dumas C, Lascu I, Morera S, Glaser P, Fourme R, Wallet V, Lacombe ML, Veron M, Janin J (1992) X-ray structure of nucleoside diphosphate kinase. EMBO J 11, 3203-3208.

Elbashir SM, Harborth J, Lendeckel W, Yalcin A, Weber K, Tuschl T. (2001) Duplexes of 21-nucleotide RNAs mediate RNA interference in cultured mammalian cells. Nature 411, 494-498.

Fulda S, Lutz W, Schwab M, Debatin KM (1999) MycN sensitizes neuroblastoma cells for drug-induced apoptosis. Oncogene 18, 1479-1486.

Guo QM, Malek RL, Kim S, Chiao C, He M, Ruffy M, Sanka K, Lee NH, Dang CV, Liu ET (2000) Identification of c-myc responsive genes using rat cDNA microarray. Cancer Res 60, 5922-5928.

Hall A (1998) G proteins and small GTPases: distant relatives keep in touch. Science 280, 2074-2075.

Koomagi R, Sauerbrey A, Zintl F, Volm M (1998) nm23-H1 protein expression in newly diagnosed and relapsed childhood acute lymphoblastic leukemia. Anticancer Res 18, 4307-4309.

Lal A, Lash AE, Altschul SF, Velculescu V, Zhang L, McLendon RE, Marra MA, Prange C, Morin PJ, Polyak K, Papadopoulos N, Vogelstein B, Kinzler KW, Strausberg RL, Riggins GJ (1999) A public database for gene expression in human cancers. Cancer Res 59, 5403-5407.

Leone A, Seeger RC, Hong CM, Hu YY, Arboleda MJ, Brodeur GM, Stram D, Slamon DJ, Steeg PS. (1993) Evidence for nm23 RNA overexpression, DNA amplification and mutation in aggressive childhood neuroblastomas. Oncogene 8, 855-865.

Lutz W, Stöhr M, Schürmann J, Wenzel A, Löhr A, Schwab M. (1996). Conditional expression of N-*myc* in human neuroblastoma cells increases expression of α-prothymosin and ornithine decarboxylase and accelerates progression into S-phase early after mitogenic stimulation of quiescent cells. Oncogene 13, 803-812.

Florenes VA, Aamdal S, Myklebost O, Maelandsmo GM, Bruland OS, Fodstad O (1992) Levels of nm23 messenger RNA in metastatic malignant melanomas: inverse correlation to disease progression. Cancer Res 52, 6088-6091.

Fritz G, Just I, Kaina B (1999) Rho GTPases are over-expressed in human tumors. Int J Cancer 81, 682-687.

Godfried MB, Veenstra M, v Sluis P, Boon K, v Asperen R, Hermus MC, v Schaik BD, Voute TP, Schwab M, Versteeg R, Caron HN (2002) The N-myc and c-myc downstream pathways include the chromosome 17q genes nm23-H and nm23-H2. Oncogene 211, 2097-101

Henriksson M, Luscher B (1996). Proteins of the Myc network: essential regulators of cell growth and differentiation. Adv Cancer Res 68, 109-182

Hennessy C, Henry JA, May FE, Westley BR, Angus B, Lennard TW (1991) Expression of the antimetastatic gene nm23 in human breast cancer: an association with good prognosis. J Natl Cancer Inst 83, 281-285.

Kozma R, Ahmed S, Best A, Lim L (1995) The Ras-related protein Cdc42Hs and bradykinin promote formation of peripheral actin microspikes and filopodia in Swiss 3T3 fibroblasts. Mol Cell Biol 15 ,1942-1952.

Leone A, Seeger RC, Hong CM, Hu YY, Arboleda MJ, Brodeur GM, Stram D, Slamon DJ, Steeg PS (1993) Evidence for nm23 RNA overexpression, DNA amplification and mutation in aggressive childhood neuroblastomas. Oncogene 8, 855-865.

Ma A, Moroy T, Collum R, Weintraub H, Alt FW, Blackwell TK (1993). DNA binding by N- and L-Myc proteins. Oncogene 8, 1093-1098.

Malynn BA, de Alboran IM, O'Hagan RC, Bronson R, Davidson L, DePinho RA, Alt FW (2000) N-myc can functionally replace c-myc in murine development, cellular growth, and differentiation. Genes Dev 14, 1390-1399.

Marcu KB, Bossone SA, Patel AJ (1992). Myc function and regulation. Annu Rev Biochem 61, 809-60.

Nesi G, Palli D, Pernice LM, Saieva C, Paglierani M, Kroning KC, Catarzi S, Rubio CA, Amorosi A (2001) Expression of nm23 gene in gastric cancer is associated with a poor 5-year survival. Anticancer Res 21, 3643-3649.

Niitsu N, Okamoto M, Okabe-Kado J, Takagi T, Yoshida T, Aoki S, Honma Y, Hirano M (2001) Serum nm23-H1 protein as a prognostic factor for indolent non-Hodgkin's lymphoma. Leukemia 15, 832-839.

Oda Y, Naka T, Takeshita M, Iwamoto Y, Tsuneyoshi M (2000) Comparison of histological changes and changes in nm23 and c-MET expression between primary and metastatic sites in osteosarcoma: a clinicopathologic and immunohistochemical study. Hum Pathol 31, 709-716.

Okabe-Kado J, Kasukabe T, Honma Y (1998) Differentiation inhibitory factor Nm23 as a prognostic factor for acute myeloid leukemia. Leuk Lymphoma 32, 19-28.

Otsuki Y, Tanaka M, Yoshii S, Kawazoe N, Nakaya K, Sugimura H (2001) Tumor metastasis suppressor nm23H1 regulates Rac1 GTPase by interaction with Tiam1. Proc Natl Acad Sci USA 98, 4385-4390.

Qiu RG, Abo A, McCormick F, Symons M (1997) Cdc42 regulates anchorage-independent growth and is necessary for Ras transformation. Mol Cell Biol 17, 3449-3458.

Pavelic K, Kapitanovic S, Radosevic S, Bura M, Seiwerth S, Pavelic LJ, Unusic J, Spaventi R (2000) Increased activity of nm23-H1 gene in squamous cell carcinoma of the head and neck is associated with advanced disease and poor prognosis. J Mol Med 78, 111-118

Ravazoula P, Aletra C, Kourounis G, Ladopoulos I, Tzigounis V (2000) Immunohistochemical analysis of nm23-H1 expression in human cervical lesions. Eur J Gynaecol Oncol 21, 510-512.

Sarner S, Kozma R, Ahmed S, Lim L (2000) Phosphatidylinositol 3-kinase, Cdc42, and Rac1 act downstream of Ras in integrin-dependent neurite outgrowth in N1E-115 neuroblastoma cells. Mol Cell Biol 20, 158-172.

Sato Y, Tsuchiya B, Urao T, Baba H, Shiku H, Kodama T, Kameya T. (2000) Semiquantitative immunoblot analysis of nm23-H1 and -H2 isoforms in adenocarcinomas of the lung: prognostic significance. Pathol Int 50, 200-205.

Schmidt EV (1999). The role of c-myc in cellular growth. Oncogene 18, 2988-2996

Schmitz AA, Govek EE, Bottner B, Van Aelst L (2000) Rho GTPases: signaling, migration, and invasion. Exp Cell Res 261, 1-12.

Schneider J, Pollan M, Jimenez E, Marenbach K, Martinez N, Volm M, Marx D, Meden H (2000) nm23-H1 expression defines a high-risk subpopulation of patients with early-stage epithelial ovarian carcinoma. Br J Cancer 82, 1662-1670.

Schuhmacher M, Kohlhuber F, Holzel M, Kaiser C, Burtscher H, Jarsch M, Bornkamm GW, Laux G, Polack A, Weidle UH, Eick D (2001) The transcriptional program of a human B cell line in response to Myc. Nucleic Acids Res 29, 397-406

Schwab M, Alitalo K, Klempnauer KH, Varmus HE, Bishop JM, Glibert F, Brodeur GM, Golsdstein M Trent, J.M. (1983). Amplified DNA with limited homology to myc cellular oncogene is shared by human nueroblastoma cell lines and a neuroblastoma tumour. Nature 305, 245-248.

Seeger RC, Brodeur GM, Sather H, Dalton A, Siegel SE, Wong KY, Hammond D (1985). Association of multiple copies of the N-myc oncogene with rapid progression of neuroblastomas. N Engl J Med 318, 1111-1116.

Stam JC, Michiels F, van der Kammen RA, Moolenaar WH, Collard JG (1998) Invasion of T-lymphoma cells: cooperation between Rho family GTPases and lysophospholipid receptor signaling. EMBO J 17, 4066-4074.

Steeg PS, Bevilacqua G, Kopper L, Thorgeirsson UP, Talmadge JE, Liotta LA, Sobel ME. (1988) Evidence for a novel gene associated with low tumor metastatic potential. : J Natl Cancer Inst 80 , 200-204.

Volm M, Mattern J, Koomagi R. (1998) Association between nm23-H expression, proliferation and apoptosis in non-small cell lung carcinomas. Clin Exp Metastasis 16, 595-602

Weiss WA, Aldape K, Mohapatra G, Feuerstein BG, Bishop JM (1997). Targeted expression of MYCN causes neuroblastoma in transgenic mice. EMBO J 16, 2985-2995.

Zhu J, Tseng YH, Kantor JD, Rhodes CJ, Zetter BR, Moyers JS, Kahn CR (1999) Interaction of the Ras-related protein associated with diabetes rad and the putative tumor metastasis suppressor NM23 provides a novel mechanism of GTPase regulation. Proc Natl Acad Sci USA96, 14911-14918.

## Claims

1. Method for the treatment of cancer by changing the intracellular activity of cdc42.

2. Method according to claim 1 wherein the activity of cdc42 is increased by administration of small molecules.

3. Method according to claim 2 wherein the small molecules are non-hydrolyzable GTP analogues of cdc-42.

4. Method according to claim 1 wherein the activity of cdc-42 is increased by inhibition of nm23-H1 and/or nm23-H2.

5. Method according to claim 4 wherein the inhibition of nm23-H1 and/or nm23-H2 is established by small molecules.

6. Method according to claim 4 wherein the inhibition of nm23-H1 and/or nm23-H2 is established by a protein which is able to bind to the nm23 protein.

7. Method according to claim 6 wherein the protein that inhibits nm23-H1 and/or nm23-H2 is an antibody or at least a binding part of an antibody.

8. Method according to claim 4 wherein the inhibition of nm23-H1 and/or nm23-H2 is established by inhibition of the expression of the genes coding for nm23-H1 and/or nm23-H2.

9. Method according to claim 1 wherein the activity of cdc-42 is decreased.

10. Method according to claim 9 wherein the activity of cdc-42 is decreased by a small molecule.

11. Method according to claim 9 wherein the activity of cdc-42 is decreased by inhibition of the expression of the gene coding for cdc-42.

12. Method according to claim 8 or claim 11 wherein the inhibition of the expression is effected by a double-stranded RNA through RNA interference.

13. Method according to claim 12 wherein the double stranded RNA comprises at least 12 nucleotides, more preferable 15 or more nucleotides, more preferably, 15-25 nucleotides, most preferably 21 nucleotides.

14. Method according to claim 8 or claim 11 wherein the inhibition of the expression is effected by an antisense nucleic acid.

15. Method according to any of claims 1-15 for the treatment or prophylaxis of cancer.

16. Method according to claim 15 wherein the cancer is selected from the group consisting essentially of neuroblastoma, colon carcinoma, gastric cancer, ovarian carcinoma, endometrial carcinoma, cervical carcinoma, sq. c. carcinoma in head and neck, non-SCLC, adenocarcinoma of the lung, osteosarcoma, retinoblastoma, childhood ALL, AML and non-Hodgkin lymphoma.

17. A compound for prophylactic and/or therapeutic treatment of an individual against cancer which is able to influence the intracellular activity of cdc-42.

18. A compound according to claim 17 which is able to increase the intracellular activity of cdc-42.

19. A compound according to claim 18 wherein the compound is a small molecule.

20. A compound according to claim 19 wherein the small molecule is a non-hydrolyzable GTP-analogue of cdc-42.

21. A compound according to claim 18 which is able to inhibit the activity of nm23-H1 and/or nm23-H2.

22. A compound according to claim 17 which is able to decrease the intracellular activity of cdc-42.

23. A compound according to claim 21 or 22 wherein the compound is a small molecule.

24. A compound according to claim 21 or 22 wherein the compound is a protein, more preferably an antibody or at least a binding part of an antibody.

25. A compound according to claim 21 or 22 wherein the compound is a nucleic acid.

26. A compound according to claim 25 wherein the compound is a double-stranded RNA.

27. A compound according to claim 25 wherein the compound is an antisense nucleic acid.

28. A compound according to claim 26 or 27 which comprises at least 12 nucleotides, more preferably 15 or more nucleotides, more preferably 15-25 nucleotides, most preferably 21 nucleotides.

29. A compound according to any of claims 17 to 28 wherein the cancer is selected from the group consisting essentially of neuroblastoma, colon carcinoma, gastric cancer, ovarian carcinoma, endometrial carcinoma, cervical carcinoma, sq. c. carcinoma in head and neck, non-SCLC, adenocarcinoma of the lung, osteosarcoma, retinoblastoma, childhood ALL, AML and non-Hodgkin lymphoma.

30. A pharmaceutical preparation comprising a compound according to any of claims 17 to 29 and a pharmaceutically acceptable incipient.

31. Use of a compound according to any of claims 17 to 29 for the preparation of a medicament for the treatment of cancer.


SEQUENCE LISTING

<110>  Academisch Ziekenhuis bij de Universiteit van Amsterdam

<120>  Novel cancer therapies

<130>  P60609EP00

<140>  EP 02077397.4
<141>  2002-06-17

<160>  14

<170>  PatentIn version 3.1

<210>  1
<211>  6
<212>  DNA
<213>  Artificial Sequence

<220>
<223>  E-box motive
<400>  1
cacgtg                                                                  6

<210>  2
<211>  36
<212>  DNA
<213>  Artificial Sequence

<220>
<223>  oligonucleotide used for Cdc42flag constructs
<400>  2
tatatagaat tcatttcagc aatgcagaca attaag                                36

<210>  3

<211>  30

<212>  DNA

<213>  Artificial Sequence

<220>

<223>  oligonucleotide used for Cdc42flag constructs

<400>  3
tatatactcg agtagcagca cacacctgcg                                        30

<210>  4

<211>  43

<212>  DNA

<213>  Artificial Sequence

<220>

<223>  oligonucleotide used for Cdc42flag constructs

<400>  4
gtgtaggata tcaggagaca tgttttacca acagcaacat cgc                         43

<210>  5

<211>  29

<212>  DNA

<213>  Artificial Sequence

<220>

<223>  oligonucleotide used for Cdc42flag constructs

<400>  5
gtgtaggata tcaggagaca gtttttacc                                         29

<210>  6

<211>  17

<212>  DNA

<213>  Artificial Sequence

<220>

<223>  oligonucleotide used for Cdc42flag constructs

<400>  6
attaaccctc actaaag                                                      17

<210> 7

<211> 21

<212> DNA

<213> Artificial Sequence

<220>

<223> sense oligonucleotide

<400> 7
caccaaggcu gucaccacat t 21

<210> 8

<211> 21

<212> DNA

<213> Artificial Sequence

<220>

<223> sense oligonucleotide

<400> 8
aggauuccgc cuuguuggut t 21

<210> 9

<211> 21

<212> DNA

<213> Artificial Sequence

<220>

<223> sense oligonucleotide

<400> 9
cagggaagaa uggucgguct t 21

<210> 10

<211> 21

<212> DNA

<213> Artificial Sequence

<220>

<223>  sense oligonucleotide

<400>  10
cuaugcaguc acaguuaugt t                                                21

<210>  11

<211>  21

<212>  DNA

<213>  Artificial Sequence

<220>

<223>  antisense oligonucleotide

<400>  11
uguggugaca gccuuggugt t                                                21

<210>  12

<211>  21

<212>  DNA

<213>  Artificial Sequence

<220>

<223>  antisense oligonucleotide

<400>  12
accaacaagg cggaauccut t                                                21

<210>  13

<211>  21

<212>  DNA

<213>  Artificial Sequence

<220>

<223>  antisense oligonucleotide

<400>  13
gaccgaccau ucuucccugt t                                                21

<210>  14

<211>  21

<212>  DNA

<213>  Artificial Sequence

<220>

<223>  antisense oligonucleotide

<400>  14
cauaccugug acugcauagt t                                                21